# EUROPÄISCHE PATENTANMELDUNG

(11) **EP 4 523 642 A1**
(43) Veröffentlichungstag der Anmeldung: **19.03.2025**
(21) Anmeldenummer: 23198030.1
(22) Anmeldetag: 18.09.2023
(51) Int. Cl.: A61B 17/86, A61B 50/30

(54) **ELASTISCH AUFWEITBARE STERILISIERBARE SCHÜTTVORRICHTUNG**

(71) Anmelder: Rösler IP GmbH, 88138 Hergensweiler (DE)
(72) Erfinder: Rösler, Thiemo, 88239 Wangen/Neuravensburg (DE)
(74) Vertreter: Riebling, Peter

(57) **Zusammenfassung**

Elastische aufweitbare Verpackung (1, 20) für kleinteilige Gegenstände (17) im medizinischen Bereich, bestehend aus einem einseitig offenen Verpackungskörper (1, 20) mit einem bodenseitig geschlossenen Verpackungsraum (25, 25'), der an seiner oberen Seite in ein elastisch aufweitbares Verschlussteil (6) übergeht, wobei im Übergangsbereich zwischen dem Verschlussteil (6) und dem Verpackungsraum (25, 25') ein oder mehrere Sperrnoppen (11, 12) angeordnet sind, die zwischen sich einen elastisch aufweitbaren Verschlussspalt (13, 13') bilden.

## Beschreibung

Gegenstand der Erfindung ist eine elastisch aufweitbare sterilisierbare Verpackung für kleinteilige Gegenstände aus dem medizinischen Bereich nach dem Oberbegriff des Patentanspruchs 1.

Die elastisch aufweitbare Verpackung dient als Verpackung für sterile medizinische Kleinteile und Ersatzteile, die insbesondere in der Chirurgie, der Orthopädie und weiteren medizinischen Bereichen verwendet werden. Solche Gegenstände müssen während der Operation - möglichst einhändig - einfach, störungsfrei und unter Beibehaltung der Sterilität aus der Verpackung entnehmbar sein.

Zweck einer Verpackung nach dem Stand der Technik und der vorliegenden Erfindung ist die Bereitstellung einer sterilisierbaren Verpackung für kleinteilige Gegenstände gleich welcher Art aus dem medizinischen Bereich, die bevorzugt durch Strahlung sterilisierbar ist.

Aus der DE 10 2013 019 452 B4 ist eine elastisch aufweitbare Einzelverpackung für langgestreckte Gegenstände bekannt geworden, wobei der Gegenstand aus mindestens einem Kopf vergrößerten Durchmessers und einem sich mit vermindertem Durchmesser anschließenden Bolzenteil besteht. Die Einzelverpackung ist aus einer mindestens einseitig stirnseitig offenen, etwa zylindrischen Verpackungshülse gebildet, die aus einem elastisch biegbaren Material besteht, die eine obere Einführöffnung zum Einführen oder Entnehmen des langgestreckten Gegenstandes aufweist, wobei lediglich der Kopf des zu haltenden Gegenstandes im Bereich der Einführöffnung der Verpackung lagengesichert gehalten wird.

Als nachteilig wird angesehen, dass die bekannte Einzelverpackung auf die Halterung des Kopfes eines langgestreckten Gegenstandes - z.B. einer Schraube - angewiesen ist, was den Anwendungsbereich stark einschränkt. Andere Gegenstände, die einen solchen Kopf mit vergrößertem Durchmesser nicht aufweisen, können nicht lagengesichert gehalten werden.

Weiterer Nachteil ist, dass die Einzelverpackung individuell an den Durchmesser des Schraubenkopfes angepasst werden muss, was dazu führt, dass eine Vielzahl von Einzelverpackungen hergestellt werden müssen, die an unterschiedliche Schraubenformen und Schraubenlängen sowie Kopfdurchmesser angepasst sind.

Es ist nur ein geringflächiger Griffbereich vorhanden, der nur im Bereich des aufweitbaren Halses der Einzelverpackung vorhanden ist, was dazu führt, dass die Griffflächen klein und dadurch schwer zu betätigen sind.

Nachdem der Schraubenkopf der zu haltenden Schraube im Bereich der Einführöffnung liegt, besteht die Gefahr, dass ein Bediener in der sterilen Umgebung des Operationssaals den Schraubenkopf unbeabsichtigt berührt und damit kontaminiert, was zu schweren Operationsschäden führen kann.

Bei der bekannten Einzelverpackung bestand demnach eine erhöhte Gefahr der Kontamination, weil der Schraubenkopf von der oberen Einführöffnung her zugänglich war, was zu vermeiden ist.

Bei der bekannten Einzelverpackung kann nur jeweils ein einziger langgestreckter Gegenstand verpackt werden, wobei vorausgesetzt ist, dass ein Schraubenkopf oder ein Kopf erweiterten Durchmessers vorhanden sind, weil die Haltefunktion in diesem Bereich stattfindet. Es handelte sich daher nur um einen etwa linienförmigen, elastisch aufweitbaren Klemmbund, der die Haltefunktion verwirklichte. Die Betätigung dieses Klemmbundes war - mangels ausreichender Griffflächen - ungünstig.

Im Übrigen ist die bekannte Einzelverpackung darauf angewiesen, dass eine bestimmte Kopfform vorgegeben ist, andernfalls die Verpackung nicht funktioniert. Daher kann auch mit der bekannten Einzelverpackung nur jeweils ein einziger Gegenstand verpackt werden und nicht mehrere schüttbare Gegenstände gleichzeitig, die möglichst vor Berührungen geschützt werden sollen.

Der Erfindung liegt deshalb die Aufgabe zugrunde, eine Verpackung für schüttfähige Gegenstände der eingangs genannten Art so weiterzubilden, dass die zu verpackenden Gegenstände betriebssicher in einer elastisch aufweitbaren Verpackung eingebracht werden können, und dass diese Gegenstände gegen unbeabsichtigte Berührung geschützt sind.

Zur Lösung der gestellten Aufgabe ist die Erfindung durch die technische Lehre des unabhängigen Patentanspruchs gekennzeichnet.

Dabei wird es bevorzugt, wenn im Übergangsbereich zwischen dem Verschlussteil und dem Verpackungsraum ein oder mehrere Sperrnoppen angeordnet sind, die miteinander einen elastisch aufweitbaren Verschlussspalt bilden.

Es handelt sich demnach um eine flache Verpackungstasche, in deren Verpackungsraum ein oder mehrere Gegenstände hineingeschüttet sind und die durch Fingerdruck auf die Griffflächen über den sich dann öffnenden Verschlussspalt entnommen werden können.

Die Entnahme kann dadurch erfolgen, dass die Schüttverpackung auf den Kopf gestellt wird und danach der Verschlussspalt durch Fingerdruck auf die Griffflächen geöffnet wird, wonach ein oder mehrere Gegenstände aufgrund der Schwerkraft herausfallen.

In einer anderen Variante kann der Gegenstand bei aufrechter Verpackung und geöffnetem Verschlussspalt mit einem Magnetwerkzeug oder einem Greifwerkzeug aus dem Verpackungsraum entnommen werden.

Die Anordnung von Sperrnoppen, die den Verpackungsraum nach oben hin begrenzen und den Verschlussspalt ausbilden hat den Vorteil, dass bei einer Fehlbedienung, wenn beispielsweise die medizinische Hilfskraft die Verpackung Überkopf umgedreht hält und entleeren will, vermieden wird, dass die im Verpackungsraum gelagerten Gegenstände unbeabsichtigt herausfallen solange die Sperrnoppen nicht betätigt sind. Die erst durch Fingerdruck zu betätigenden Sperrnoppen, die den Verschlussspalt bilden, sind eine Sicherung gegen unbeabsichtigtes Entleeren.

Dabei wird es bevorzugt, wenn insgesamt ein elastisch aufweitbarer, flacher Verpackungskörper vorhanden ist, dessen oberes Verschlussteil elastisch aufweitbar ist, wobei das Verschlussteil durch mindestens zwei auf den Schmalseiten des Verpackungskörpers gegenüber liegend angeordnete Griffrippen gebildet ist, die elastisch gegeneinander gedrückt werden können, wodurch der im unteren Bereich des Verschlussteils angeordnete, wodurch sich der durch ein oder mehrere Sperrnoppen gebildete Verschlussspalt aufweitet.

Durch den aufgeweiteten Verschlussspalt können die in den Verpackungsraum geschütteten Gegenstände aus der Verpackung herausgeschüttet werden, wenn diese mit der Entnahmeöffnung nach unten gerichtet ist.

Dabei wird es bevorzugt, wenn die ein oder mehreren Sperrnoppen jeweils innenliegend auf der Vorderwand und der Rückwand des Verschlussteils gegeneinander gerichtet und zueinander fluchtend angeordnet sind, wodurch sie sich bei geeignetem Verformungsdruck auf die einander gegenüberliegenden Griffflächen voneinander entfernen und dadurch einen geöffneten Verschlussspalt bilden, durch den hindurch der Gegenstand eingebracht oder entnommen werden kann. Jede Sperrnoppe ist ein etwa punkt- oder knopfförmiges Teil, welches jeweils an der Innenseite der Vorder- und Rückwand angeformt ist und das etwa rund- oder oval oder eckig oder polygonal profiliert ist.

Nach der Erfindung wird es demnach bevorzugt, wenn der obere Verschlussteil des Verpackungskörpers durch eine durch Fingerkraft aufweitbare Öffnung begrenzt ist, und dass unterhalb dieser Öffnung ein oder mehrere gegenüberliegende, jeweils fluchtend auf der Vorder- und der Rückwand angeordnete Sperrnoppen angeordnet sind, die zwischen sich einen aufweitbaren Verschlussspalt bilden.

Mit der gegebenen technischen Lehre ergibt sich der Vorteil, dass die zu verpackenden Gegenstände nicht mehr in notwendiger Weise eine Schraube mit einem im Durchmesser vergrößerten Schraubenkopf sein müssen, sondern sie können beliebig geformte Gegenstände sein, die auch im Wege eines Schüttvorganges durch die im oberen Bereich aufgeweiteten Öffnung und durch den darunter liegenden aufgeweiteten Verschlussspalt hindurch in den unteren Verpackungsraum des Verpackungskörpers gelangen, ohne dass im oberen Bereich der Öffnung des Verpackungskörpers eine Berührungsgefahr besteht.

Damit können verbesserte sterile Anforderungen erfüllt werden und es können ein oder mehrere Gegenstände in einem solchen Verpackungskörper sicher verpackt werden, die bei geschlossenem Verschlussspalt berührungssicher im Verpackungsbereich aufbewahrt werden können, ohne dass es auf lagengesicherte Halterung eines einzigen Gegenstandes ankommt.

Hier unterscheidet sich die Erfindung vom Stand der Technik nach der DE10 2013 019 452 B4, denn die bekannte Verpackung war nur für die Halterung einer einzigen Schraube vorgehen und die Form der Verpackung musste an die Form des zu haltenden Gegenstandes angepasst werden. Bei der Erfindung ist dies nicht mehr notwendig. Damit entfällt auch die Notwendigkeit, eine Vielzahl von Verpackungsvarianten für eine Vielzahl von unterschiedlichen, zu verpackenden Gegenständen bereitzustellen, weil in einer einzigen Verpackung eine Vielzahl von - auch unterschiedlichen - kleinteiligen Gegenständen verpackt werden können, die zum Beispiel Stifte, Schrauben, Muttern, Platten oder anderen Verpackungsgegenstände sein können, die bei aufgeweiteter Öffnung der Verpackung durch den im Abstand unter der Verpackungsmündung angeordneten, aufweitbaren Verschlussspalt hindurchpassen und bei geschlossenem Verschlussspalt nicht mehr die Verpackung verlassen können.

Die Zuhaltekraft für die den Verschlussspalt bildenden Sperrnoppen wird durch die elastische Rückstellkraft des Kunststoffmaterials im Halsbereich der Verpackung definiert.

In einer anderen Ausgestaltung der Erfindung kann es noch zusätzlich vorgesehen sein, dass die Zuhaltekraft (Schließkraft) des Verschlussspaltes dadurch vergrößert ist, dass die Sperrnoppen einen Verzahnungseingriff bilden.

Weil durch die technische Lehre die Erfindung es nun erstmals möglich ist, einen geschützten Verpackungsraum in einer Schüttverpackung zu bilden, indem ein oder mehrere Gegenstände sicher aufbewahrt werden können, ergibt sich der weitere Vorteil, dass es im oberen Bereich, das heißt jenseits der Sperrnoppen und demnach im Bereich des oberen Randes erstmals möglich ist, die gesamte Öffnung durch Verschlussmittel sicher zu verschließen.

Ein erster sicherer Verschluss des Öffnungsrandes ist beispielsweise, dass das Kunststoffmaterial, aus dem der Verpackungskörper besteht, im Wege einer Verschweißung verschweißt werden kann, um so eine absolut dichte Verpackung zu schaffen, was bei der bekannten Einzelverpackung nach dem Stand der Technik nicht möglich war.

Eine weitere Verschlussmöglichkeit ist zum Beispiel, dass die Öffnung des Verpackungskörpers im oberen Bereich des umlaufenden Randes mit einem gesiegelten und abreißbaren Verschlussblatt oder mit einem Verschlusskleber verschlossen werden kann.

Wichtig bei der Erfindung ist jedenfalls, dass jenseits und unterhalb der Sperrnoppen ein in sich geschlossener Verpackungsraum gebildet ist, und dass der oberhalb der Sperrnoppen befindliche Halsbereich des Verpackungskörpers in beliebiger Weise abgedichtet verschlossen werden kann, wobei insbesondere eine luftdichte Versiegelung, zum Beispiel durch Verschweißung bevorzugt wird.

Nachdem es sich um eine Verpackung für sterilisierbare Gegenstände handelt, werden solche Verpackungskörper bevorzugt im medizinischen Bereich eingesetzt und die zu verpackenden Gegenstände sind bevorzugt medizinische, sterilisierbare Gegenstände, wie zum Beispiel Schrauben, Noppen, Muttern und Stifte und dergleichen mehr. Die Sterilisierung erfolgt bevorzugt an der mit Verpackungsgegenständen befüllten und dicht verschlossenen Verpackung im Wege einer Strahlungssterilisierung.

In einer anderen bevorzugten Ausführung ist vorgesehen, dass die elastisch aufweitbare Schüttverpackung in eine luftdichte Beutelverpackung gesteckt wird und danach sterilisiert wird.

Der Verpackungskörper soll gut handhabbar sein. Er soll bevorzugt mit den Fingern einer Hand betätigt werden. Daher liegen die bevorzugten Dimensionen der Verpackung im Bereich einer Länge von z. B. 40 mm bis zu einer Länge von 400 mm. Eine bevorzugte Breite ist etwa 26 mm bis zu einer bevorzugten Breite von etwa 200 mm.

Es handelt sich hierbei lediglich um bevorzugte Größenabmessungen, welche den Erfindungsbereich der Erfindung nicht beschränken.

Charakteristikum der erfindungsgemäßen Verpackung ist ferner, dass es sich um einen aus Kunststoff bestehenden, vorzugsweise transparenten - oder auch leicht eingefärbten - elastisch verformbaren Flachkörper handelt, das heißt, um einen Körper, der aus zwei zueinander parallelen Wänden besteht, nämlich einer Rückwand und einer Vorderwand, und die beiden Wände parallel zueinander sind und seitlich durch schmalere Seitenwände in sich verbunden sind, wodurch sich insgesamt ein etwa flacher, taschenförmiger, im Querschnitt etwa rechteckförmiger Körper ergibt.

Eine bevorzugte Form des Verpackungskörpers ist eine Rechteckform oder eine an die Rechteckform angenäherte Form mit abgerundeten Ecken.

Eine andere Ausgestaltung sieht vor, dass die zueinander parallelen Vorder- und Rückwände eine leicht bauchige (konvexe) Form haben.

Die Rechteckform hat den Vorteil, dass die Verpackung gegen unbeabsichtigtes Wegrollen von einer Lagerfläche geschützt ist.

Die Betätigung erfolgt durch Fingerdruck auf einander gegenüberliegende Griffrippen, die im Bereich des oberen Verschlussteils angeordnet sind. Die Betätigungskraft entspricht etwa der Fingerkraft, die notwendig ist, um eine Wäscheklammer zu öffnen.

Dabei wird die etwa flache, ovale Querschnittsform des Verpackungskörpers im Halsbereich in eine angenäherte Rundform übergeführt. Dadurch weitet sich die vorher ovale Öffnung im oberen Bereich des Verschlussteils auf und die im unteren Bereich des Verschlussteils angeordneten gegeneinander gerichteten und zueinander fluchtenden Sperrnoppen, die zwischen sich den Verschlussspalt bilden, entfernen sich voneinander, wodurch sich der dazwischen liegende Verschlussspalt öffnet und ein oder mehrere Gegenstände aus dem unteren Verpackungsraum durch den geöffneten Verschlussspalt entnommen oder eingeführt werden können.

Bei größeren Verpackungskörpern ist vorgesehen, dass sowohl an der Vorderwand als auch an der Rückwand jeweils paarweise einander gegenüberliegende Sperrnoppen angeordnet sind. Das heißt die Sperrnoppen sind paarweise auf der Vorderwand und paarweise auf der Rückwand angeordnet und fluchten in einer horizontalen Linie zueinander und bilden den durch Fingerdruck oder - bei sehr großen Verpackungen - durch Maschinendruck aufweitbaren Verschlussspalt.

Der Verpackungskörper ist bevorzugt in seinem oberen Halsbereich so elastisch ausgebildet, dass die Griffrippen durch Fingerdruck gegeneinander gepresst werden können, um so eine Aufweitung der umlaufenden Öffnungsrandes zu erreichen.

Hierauf ist die Erfindung nicht beschränkt. In einer anderen Ausgestaltung kann es bei größeren Verpackungen vorgesehen sein, dass zur Befüllung oder zur Entnahme von Verpackungsgegenständen auch Maschinenhilfe zur elastischen Verformung des Halsbereichs in Anspruch genommen wird.

Bei kleineren Verpackungskörpern reicht es allerdings aus, nur ein Paar von gegenüberliegenden Sperrnoppen im unteren Teil des Halsbereichs anzuordnen. Auf jeder Verpackungsseite ist demnach nur eine Sperrnoppe vorgesehen. Bei größeren Verpackungen können auf jeder Verpackungsseite mehrere Sperrnoppen in gegenseitigem Abstand angeordnet sind, die in Lückeneingriff mit den auf der gegenüberliegenden Verpackungsseite angeordneten Sperrnoppen kommen, um so den Verschlussspalt zu bilden.

Die Formgebung der Sperrnoppen ist in weiten Grenzen variierbar. Es kann vorgesehen sein, dass die Sperrnoppen den Verschlussspalt zwar in der Verschlussstellung sicher begrenzen, andererseits aber ein Herausschütteln oder Herausgleiten des verpackenden Gegenstandes nicht behindern. Daher können die in Lückeneingriff stehenden Sperrnoppen einen gegenseitigen Abstand einnehmen. Bei dieser Variante besteht deshalb kein reibschlüssiger gegenseitiger Kontakt zwischen den Sperrnoppen.

In einer anderen Variante können die in Lückeneingriff stehenden Sperrnoppen einen gegenseitigen reibschlüssigen Kontakt haben, weil sie wie die Zähne einer Zahnreihe ineinandergreifen.

Ebenso wird es bevorzugt, wenn die paarweise angeordneten Sperrnoppen einen gegenseitigen Abstand zwischen sich bilden, weil beim Zusammendrücken eines solchen Verpackungskörpers eine Knickung oder Auswölbung der Kunststoffwand zwischen den Sperrnoppen im Bereich dieses Zwischenabstandes erfolgt und dadurch eine verbessere Aufweitung der Öffnung und des Verschlussspaltes möglich ist.

Bei einer anderen Ausgestaltung der Erfindung kann es vorgesehen sein, dass die paarweise jeweils auf der Vorderwand und der Rückwand angeordneten Sperrnoppen miteinander verbunden sind, das heißt der mittige Abstand zwischen den Sperrnoppen - der vorher den Knickbereich bildete - entfällt dann. Damit ist dafür gesorgt, dass die beiden paarweise angeordneten Sperrnoppen zu jeweils einer einzigen horizontalen langgestreckten Sperrnoppe auf der Vorderwand und der Rückwand verschmelzen, wobei immer vorausgesetzt wird, dass die Sperrnoppen auf der Vorder- und der Rückwand zueinander fluchten.

Die Erfindung ist im Übrigen nicht darauf angewiesen, dass die Sperrnoppen sowohl auf der Vorderwand als auch auf der Rückwand angeordnet sind. Es kann in einer davon abweichenden Ausgestaltung vorgesehen sein, dass entweder nur an der Innenseite der Vorderwand oder nur an der Innenseite der Rückwand einzelne oder paarweise angeordnete stift- oder punktförmige Sperrnoppen vorhanden sind, die einen Verschlussspalt zur jeweils glatten Innenseite der gegenüberliegenden Wand bilden, wodurch der Verschlussspalt dann nicht mehr mittig in dem Verpackungskörper angeordnet ist, sondern seitlich versetzt ist.

In einer weiteren Ausgestaltung der Erfindung ist vorgesehen, dass die auf der Vorderwand und der Rückwand einander gegenüberliegend angeordneten Sperrnoppen nicht zueinander fluchten, sondern auf einer horizontalen Linie seitlich gegeneinander versetzt sind, sodass sie eine in sich ineinandergreifende Zahnreihe bilden.

Die auf Abstand an der Vorderwand angeordneten Sperrnoppen greifen dann in der Art eines Zahneingriffes in die Zwischenräume der auf der Rückwand angeordneten Sperrnoppen ein und bilden so einen verzahnten, labyrinthartigen Verschlusspalt bilden, der dadurch eine besondere Verschlusscharakteristik hat. Der Verzahnungseingriff kann in einer ersten Ausführung ein gegenseitiges Spiel zwischen den ineinandergreifenden Sperrnoppen erlauben. In einer zweiten Ausführung kann der Verzahnungseingriff auch reibschlüssig sein, das heißt, die ineinandergreifenden "Zähne" der Sperrnoppen bilden eine zusätzliche Verschlusskraft aus, deren Betrag über die elastische Rückstellkraft des Kunststoffs im Halsbereich hinausgeht.

Die paarweise gegeneinander gerichteten, einen gegenseitigen Zahneingriff bildenden Sperrnoppen müssen nicht nur paarweise vorhanden sein, sondern es reicht in einer anderen Variante der Erfindung aus, dass eine Sperrnoppe an der Vorderseite und eine gegenüberliegende Sperrnoppe an der Rückseite angeordnet ist und die beiden Sperrnoppen aber nicht zueinander fluchten, sondern seitlich zueinander versetzt sind, um so ebenfalls einen Zahneingriff zu ermöglichen und damit einen abgeknickten Verschlussspalt bilden.

Während also die ersten Ausführungsformen, die bevorzugt in den Zeichnungen beschrieben sind, einen horizontalen, gerade durchlaufenden Verschlusspalt beschreiben, sind in den soeben beschriebenen Varianten die Sperrnoppen so angeordnet, dass sie einen gegenseitigen Zahneingriff bilden, um so nicht einen langgestreckten ebenen Verschlussspalt zu bilden, sondern einen gezackten, ineinandergreifenden Verschlussspalt.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung offenbarten Angaben und Merkmale, insbesondere die in den Zeichnungen dargestellte räumliche Ausbildung, könnten als erfindungswesentlich beansprucht werden, soweit sie einzeln oder in Kombination gegenüber dem Stand der Technik neu sind. Die Verwendung der Begriffe "wesentlich" oder "erfindungsgemäß" oder "erfindungswesentlich" ist subjektiv und impliziert nicht, dass die so benannten Merkmale zwangsläufig Bestandteil eines oder mehrerer Patentansprüche sein müssen.

Im Folgenden wird die Erfindung anhand von lediglich einen Ausführungsweg darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: Perspektivische Ansicht einer ersten Ausführung eines Verpackungskörpers
- Figur 2:: Ein Längsschnitt durch den Verpackungskörper nach der Schnittlinie B-B in Figur 3
- Figur 3:: Draufsicht auf den Verpackungskörper aus der Richtung seiner Öffnung
- Figur 4:: Seitenansicht des Verpackungskörpers nach Figur 1 und 2
- Figur 5:: Die Vorderansicht des Verpackungskörpers
- Figur 6:: Schnitt gemäß Linie A-A in Figur 5
- Figur 7:: Die perspektivische Ansicht des Verpackungskörpers in aufgeweitetem Zustand
- Figur 8:: Der Längsschnitt durch den Verpackungskörper
- Figur 9:: Die Draufsicht auf den Verpackungskörper im aufgeweiteten Zustand
- Figur 10:: Die Seitenansicht des Verpackungskörpers
- Figur 11:: Die Vorderansicht des Verpackungskörpers
- Figur 12:: Schnitt gemäß der Linie AA in Figur 11
- Figur 13:: Schnitt durch den Verpackungskörper im geschlossenen Zustand mit einem Verpackungsgegenstand
- Figur 14:: Die gleiche Darstellung wie Figur 13 mit einem Verpackungsgegenstand in einer anderen Lage
- Figur 15:: Draufsicht auf den Verpackungskörper nach Figur 13
- Figur 16:: Draufsicht auf den Verpackungskörper nach Figur 14:
- Figur 17:: Schnitt durch den Verpackungskörper entlang einer Linie A-A nach den vorhergehenden Zeichnungen mit dem eingesetzten Verpackungskörper
- Figur 18:: Darstellung des geöffneten Verpackungskörpers beim Einführen eines Verpackungsgegenstandes in eine erste Stellung
- Figur 19:: Die gleich Darstellung wie Figur 18 in einer zweiten Verpackungsstellung
- Figur 20:: Die gleiche Darstellung ein einer dritten Verpackungsstellung
- Figur 21:: Der Schnitt durch die Verpackung entsprechend der Darstellung in Figur 20
- Figur 22:: Die Draufsicht auf den Verpackungskörper bei geöffnetem Verschlussspalt und eingeführtem Verpackungsgegenstand
- Figur 23:: Die Seitenansicht des aufgeweiteten Verpackungskörpers
- Figur 24:: Die Vorderansicht des Verpackungskörpers im aufgeweiteten Zustand
- Figur 25:: Schnitt durch den Verpackungskörper nach Linie A-A in Figur 24
- Figur 26:: Eine zweite Variante eines Verpackungskörpers mit kleineren Abmessungen im Vergleich zur ersten Variante
- Figur 27:: Der Schnitt durch den Verpackungskörper
- Figur 28:: Die Draufsicht auf den Verpackungskörper
- Figur 29:: Die Vorderansicht des Verpackungskörpers
- Figur 30:: Schnitt gemäß der Linie A-A in Figur 29
- Figur 31:: Die Darstellung des Verpackungskörpers nach Figur 26 - Figur 30 im aufgeweiteten Zustand
- Figur 32:: Der Verpackungskörper nach Figur 31 in Längsschnitt
- Figur 33:: Die Draufsicht auf den Verpackungskörper
- Figur 34:: Der Schnitt gemäß einer Linie A-A in einer der vorhergehenden Figuren
- Figur 35:: Der Verpackungskörper nach den Figuren 26 - 34 mit einem anderen Verpackungsgegenstand
- Figur 36:: Eine andere Verpackungssituation im Vergleich zur Figur 35
- Figur 37 und Figur 38:: Die Draufsicht auf die Darstellungen in Figur 35 und 36
- Figur 39:: Der Schnitt gemäß einer der Linien A-A in den vorhergehenden Zeichnungen
- Figur 40:: Die Verpackungssituation des in der Länge verkürzten Verpackungsbehälters in einer ersten Stellung
- Figur 41:: Eine zweite Stellung
- Figur 42:: Eine dritte Stellung
- Figur 43:: Schnitt durch den Verpackungskörper in der Stellung nach Figur 42
- Figur 44:: Draufsicht auf die Öffnung
- Figur 45:: Schnittansicht beim Einführen eines Verpackungsgegenstandes in den Verpackungskörper.

Der Verpackungskörper 1 nach der Erfindung hat eine im Wesentlichen flache langgestreckte Körperform, das heißt, er ist im Querschnitt etwa rechteckförmig, denn er besteht aus einer flachen Vorderwand 2, einer hierzu parallelen Rückwand 4 und zwei sich werkstoffeinstückig anschließenden kürzeren Seitenwänden 3.

Der gesamte Verpackungskörper 1 ist bevorzugt in einem Stück im Hohlblasverfahren hergestellt und besteht aus einem elastischen Kunststoff, bevorzugt einem TPU-Kunststoff. Daher ist er sterilisierbar, schweißbar und abriebfest. Er ist bevorzugt transparent oder leicht eingefärbt und dauerelastisch. Er bildet eine flache Verpackungstasche. Er hat bevorzugt eine Härte im Bereich von 45 bis 95 shore-A und dabei besonders bevorzugt eine Härte von 85 shore-A +- 5.

Die bevorzugten Abmessungen wurden bereits schon im allgemeinen Beschreibungsteil erwähnt, wobei der Verpackungskörper nach der Erfindung für die Verpackung relativ kleiner Gegenstände geeignet sein soll, wie sie im medizinischen Bereich vorkommen, wobei unter dem Begriff "kleine Gegenstände" auch Organersatzteile gehören, wie zum Beispiel künstliche Kniegelenke, Schultergelenke, Knochennägel, Hüftgelenke, Knochensägeblätter und/oder Kleinteile, wie zum Beispiel Schrauben, Muttern, Stifte, Platten und dergleichen mehr.

Die oben beschriebenen Gegenstände sollen nur grundsätzlich die größten Maße des Verpackungskörpers beschreiben. Die in den Zeichnungen dargestellten Ausführungsbeispiele zeigen hingegen bevorzugte tatsächliche Abmessungen eines Verpackungskörpers im Abbildungsmaßstab 1:1 zur sterilen Verpackung von Schrauben, Muttern, Stifte, Platten und dergleichen. Die in den Zeichnungen dargestellten Verpackungskörper sind zeichnerisch demnach in natürlicher Größe dargestellt.

Der die Gegenstände aufnehmende Verpackungsraum 25 ist langgestreckt und bodenseitig durch eine abgerundetes Bodenteil 5 abgeschlossen. Der Verpackungsraum 25 und das Bodenteil 5 sind ebenfalls elastisch zusammendrückbar und können durch Fingerdruck von ihrer flachen Form in eine der Rundform angenäherte bauchige Form elastisch verformt werden.

Diese elastische Verformbarkeit erleichtert die Entnahme von im Verpackungsraum 25 gelagerten Gegenständen 17.

Der Übergang vom ovalen Verpackungsraum 25 in den sich oben anschließenden ovalen Halsbereich 8 eines Verschlussteils 6 erfolgt über eine bogenförmige konkave Kontur. Der Halsbereich 8 ist deshalb im Querschnitt gegenüber dem Verpackungsraum 25 in seiner Breite verringert. Er bildet eine obere Öffnung 9, die durch elastische Verformung in eine der Rundform angenäherte Öffnung 9' übergeführt werden kann.

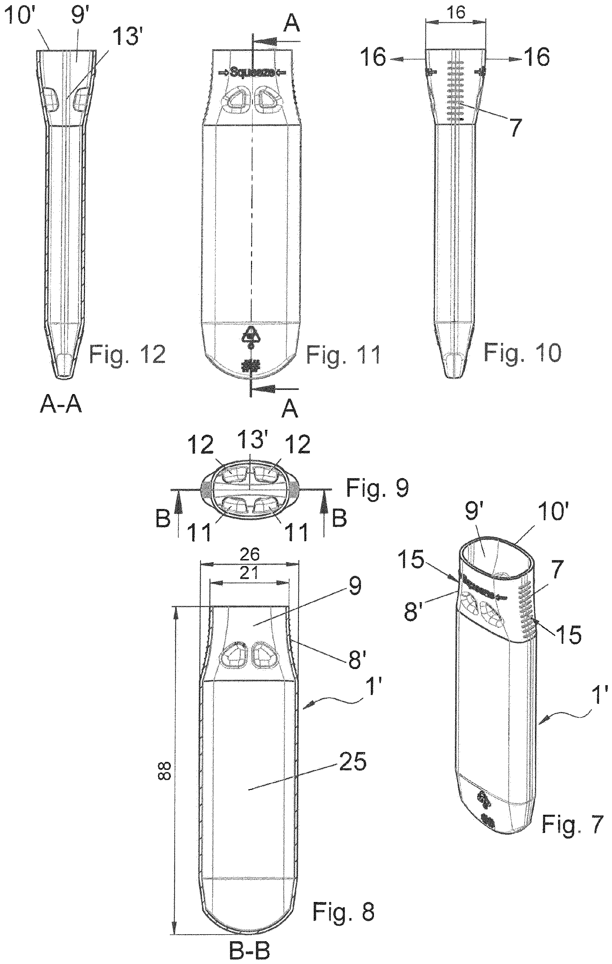

Die unverformte ovale Öffnung 9 ist durch einen umlaufenden Rand 10 definiert und im Bereich der Seitenwände 3 sind im Bereich des Verschlussteils 6 zueinander fluchtend gegenüberliegende Griffrippen 7 angeordnet, die über Fingerdruck oderbei automatischen Verpackungsmaschinen mit Maschinenhilfe - gegeneinander gequetscht werden können. Die sich hieraus ergebende Formgebung ist in den folgenden Figuren 7 ff. beschrieben.

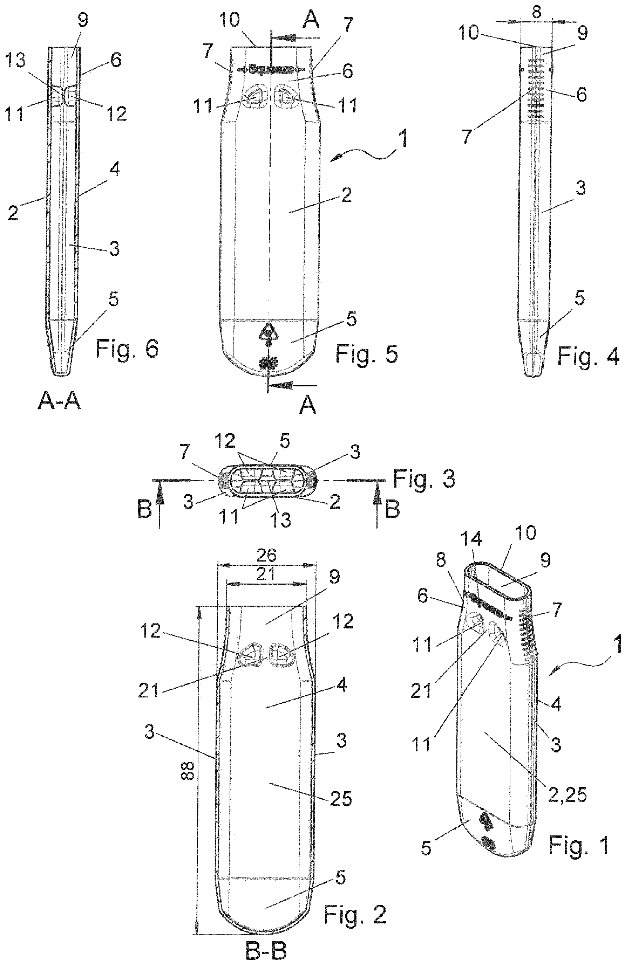

Wichtig ist, dass im unteren Bereich des Verschlussteils 6 eine Sperrmechanik angeordnet ist, die im gezeigten Ausführungsbeispiel aus paarweise gegenüberliegenden Sperrnoppen 11, 12 gebildet ist, die zueinander fluchtend angeordnet sind, wobei ein Paar der Sperrnoppen 11 auf der Innenseite der Vorderwand 2 und ein gleiches Paar von Sperrnoppen 12 auf der Innenseite der Rückwand 4 angeordnet sind und die paarweise angeordneten Sperrnoppen 11, 12 fluchtend gegenüberliegen, wie dies aus Figur 3 erkennbar ist. Sie bilden im geschlossenen Zustand demnach einen in horizontale Richtung verlaufenden Verschlussspalt 13, der im geschlossenen Zustand eine bevorzugte Weite im Bereich von 0 mm bis 1 mm haben kann.

In der allgemeinen Beschreibung wurde bereits schon darauf hingewiesen, dass es nicht lösungsnotwendig ist, dass sich die paarweise gegenüberliegend angeordneten Sperrnoppen 11, 12 gegenseitig berühren. Es kann aber in einer anderen Ausführungsform vorgesehen sein, dass die Sperrnoppen 11, 12 auf Lücke zueinander versetzt sind, um so einen verzahnten abgedichteten oder nicht abgedichteten Eingriff miteinander zu bilden.

Ebenso wurde in der allgemeinen Beschreibung schon erwähnt, dass anstatt der Anordnung von paarweisen Sperrnoppen 11, 12 auch Einzelsperrnoppen 11, 12 vorgesehen sein können.

Es können aber auch mehr als zwei Sperrnoppen jeweils an einer Innenseite der Vorderwand oder einer Innenseite der Rückwand angeordnet sein.

Demnach ist die Anzahl der Sperrnoppen, die an der Vorderwand 2 und auf der Rückwand 4 angeordnet sind, nicht beschränkt. Es können auch mehr als zwei vorhanden sein.

Es wird bevorzugt, wenn im Bereich des Verschlussteils 6 eine Beschriftung 14 angeordnet ist, die darauf hinweist, dass die Griffrippen 7 durch Fingerdruck gegeneinander komprimiert werden können, um so die Verpackung in einen geöffneten Zustand überzuführen.

Die Figuren 2-6 zeigen weitere Einzelheiten der Konstruktion, wobei aus Figur 2 noch erkennbar ist, dass zwischen den paarweise angeordneten Sperrnoppen 11, 12 jeweils ein Abstand 21 vorhanden ist. Die Anordnung des Abstandes 21 ermöglicht, dass wenn die Griffrippen 7 gegeneinander komprimiert werden, dass sich das Material im Bereich des Abstandes 21 ausbaucht und einen Knick- oder Auswölbungsbereich bildet, um so eine Verringerung der Kompressionskraft bei Öffnung des Verschlussteils 6 zu ermöglichen. Damit wird die durch Fingerdruck bewirkte Zusammendrückungskraft verringert und gleichzeitig die Weite des Verschlussspaltes 15 vergrößert. Die Zusammendrückungskraft entspricht in allen Fällen - unabhängig vom Vorhandensein eines knickbaren Abstandes 21 oder nicht - bevorzugt der Kraft, die aufgewendet werden muss, um eine Wäscheklammer zu öffnen.

In einer anderen Ausgestaltung kann es vorgesehen sein, dass die auf der Vorder- und der Rückwand paarweise angeordneten Sperrnoppen 11, 12 jeweils zu einer einzigen horizontalen Sperrnoppe umgeformt sind. Deshalb fehlt bei dieser Ausführung ein knickbarer Zwischenabstand zwischen den Sperrnoppen einer Wandseite.

Bei der Beschreibung des Verpackungskörpers ist es gleichgültig, welche Formgebung die Verpackungsgegenstände haben, wie nachfolgend gezeigt wird. Das heißt, die Verpackungsgegenstände können eine solche Länge haben, dass sie gerade im Verpackungsraum 25 oben an der Unterseite der Sperrnoppen 11, 12 und unten am Bodenteil 5 anstoßen. Sie können aber auch wesentlich kürzer sein, sodass die Verpackung die Charakteristik einer taschenförmige Schüttverpackung für die betriebssichere Lagerung von medizinischen Kleinteilen hat.

Es können somit eine Vielzahl von kleinen und kurzen Gegenständen über den geöffneten Verschlussspalt 13' in den Verpackungsraum 25 hineingeschüttet werden, um dann bei geöffnetem Verschlusspalt 13' wieder entnommen zu werden.

Die Figuren 7 bis 12 zeigen die geöffnete Verpackung, wobei vorausgesetzt wird, dass in den Pfeilrichtungen 15, 16 mit Fingerdruck oder mit maschineller Hilfe ein gegenseitiger Druck auf die Griffrippen 7 ausgeübt wird und sich die vorher rehteckförmige Öffnung 9 in eine etwa rundprofilierte Öffnung 9' aufweitet. Dabei öffnet sich auch der Verschlussspalt 13, weil sich die Sperrnoppen 11, 12 auf der Vorder- und Rückwand voneinander entfernen und einen geöffneten Verschlussspalt 13' bilden.

Gemäß Figur 9 können dann durch den geöffneten Verschlussspalt 13' hindurch die zu verpackenden Gegenstände in den Verpackungsraum 25 geschüttet werden.

Die Figur 12 zeigt, dass die Sperrnoppen 11, 12 in bestimmter Weise profiliert sind, das heißt, sie haben seitliche und gegebenenfalls auch obere und untere Einführschrägen, sodass ein besonders einfaches Einführen oder Entnehmen eines zu verpackenden Gegenstandes über die oberen und unteren Einführschrägen möglich ist.

Um eine bessere Entformung im Hohlblaswerkzeug zu ermöglichen, ist jede Sperrnoppen bevorzugt etwa kegelförmig profiliert und hat eine abgeflachte oder abgeschnittene Kegelspitze.

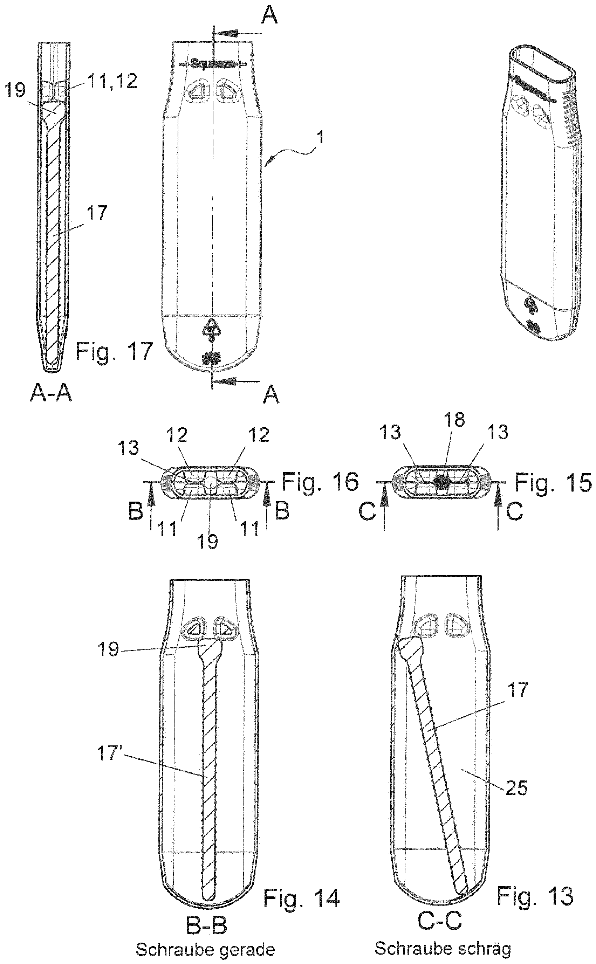

In den Figuren 13 bis 17 ist die Verpackung eines an die Verpackung angepassten Gegenstandes gezeigt, das heißt eines Gegenstandes 17, der den Verpackungsraum 25 im Wesentlichen ausfüllt.

Dabei kann der Gegenstand 17 im Verpackungsraum 25 eine schräge Stellung einnehmen wie Figur 13 zeigt oder auch eine gerade Stellung wie in Figur 14. Seine Lage im Verpackungsraum 25 ist also willkürlich. Dabei wird es bevorzugt, wenn auch der Verpackungsraum 25 durch Fingerdruck elastisch verformbar ist. Damit kann eine eventuelle Verklemmung des Gegenstandes im Verpackungsraum 25 behoben werden.

Es ist also nicht lösungsnotwendig, den Gegenstand 17 lagengesichert mit seinem Kopf an der Unterseite der Sperrnoppen 11, 12 zur Anlage zu bringen, weil er nicht auf diese Lagensicherung angewiesen ist.

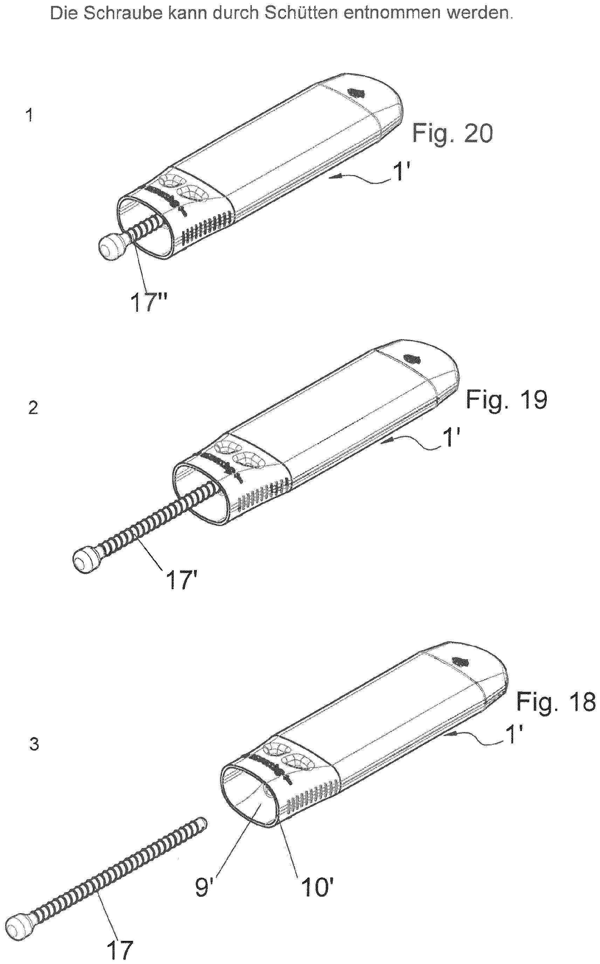

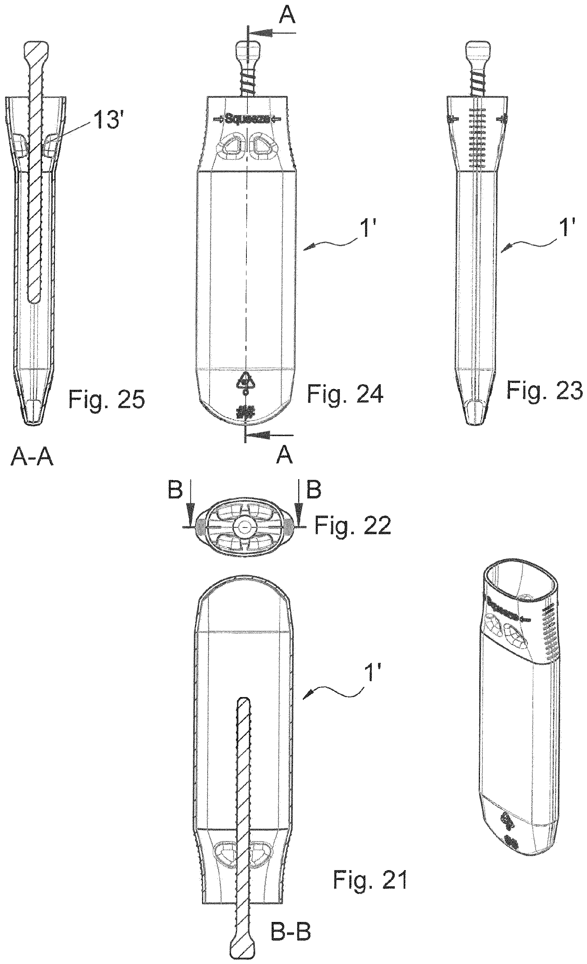

Die Figuren 21 bis 25 zeigen die Verpackung eines solchen Gegenstandes nach den Figuren 13 bis 17, wobei die Figuren 18 bis 20 das schrittweise Einführen eines solchen Gegenstandes 17 zeigen, während die Figur 21 einen Schnitt durch die Verpackung in der Lage des Gegenstandes 17" nach Figur 20 ist.

Dabei zeigt die Figur 25, dass der Gegenstand bei geöffnetem Verschlussspalt 13' eingeführt wird. Dabei kann die lichte Weite des Verschlussspaltes 13' wesentlich größer sein als der Durchmesser des Bolzens des Gegenstandes 17, sodass dieser frei fallend in den Verpackungsraum 25 hineinkommt.

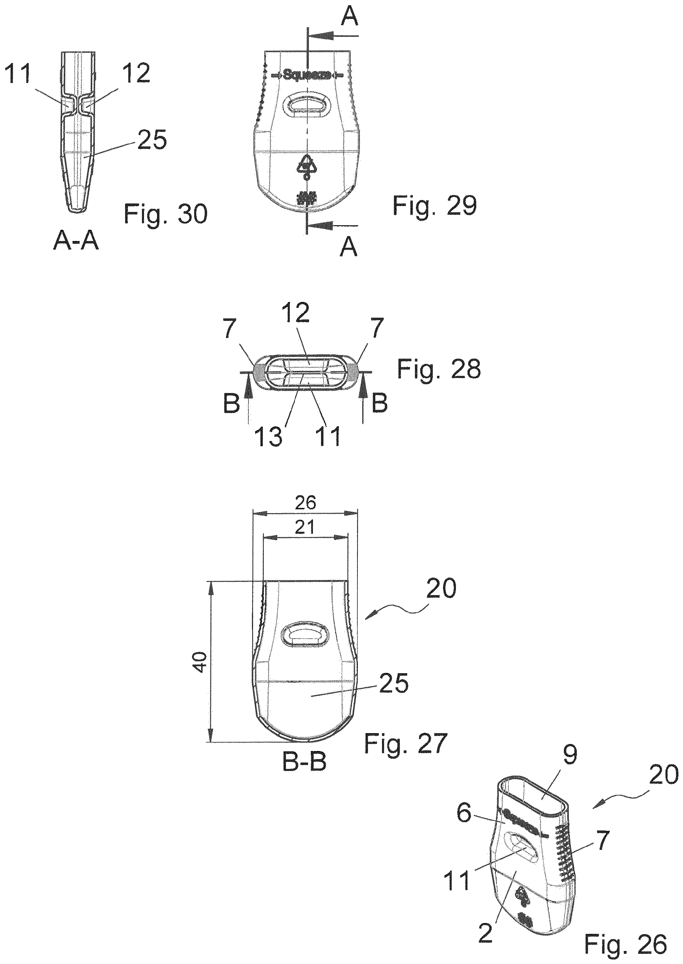

Die Figuren 26 bis 30 zeigen einen weiteren Verpackungskörper 20, der in seinen Abmessungen gegenüber dem Verpackungskörper 1 vermindert ist und der eine kürzere Länge aufweist, wobei hier jedoch die Dimensionen der Öffnung 9 im Wesentlichen gleich sind.

Kennzeichnend für diese Ausführungsformen ist, dass die elastisch aufweitbare, sterilisierbare Verpackungstasche eine kürzere Verpackungslänge mit einem im Volumen verringerten Verpackungsraum 25' aufweist, und dass im Verschlussteil 6 nicht paarweise gegeneinander gerichtete Sperrnoppen 11, 12 vorhanden sind, sondern nur eine einzelne Sperrnoppe 11, die einer einzelnen Sperrnoppe 12 gegenüberliegt, wie dies beispielsweise aus dem Schnitt in Figur 30 zu entnehmen ist.

Eine solcher Verpackungskörper 20 kann in allen beschriebenen Ausführungen auch als Verschlussstopfen einer nicht näher dargestellten hülsenförmigen, einseitig offenen Verpackung verwendet werden. Dabei kann der Verpackungskörper 1 in die Öffnung der Verpackungshülse als Verschlussstopfen abgedichtet und klemmend eingedrückt werden. Die Griffflächen bilden dann reibungserhöhende Vorsprünge, die sich an den Innenseiten der Verpackungshülse im Bereich deren Öffnung reibschlüssig anlegen.

Die aus diesen zwei Teilen bestehende Hülsenverpackung ist z.B. für die sterile Verpackung von Sägeblätter für Knochensägen in der Medizintechnik verwendbar. Die mit dem Verschlussstopfen verschlossene Hülsenverpackung kann in einen die Verpackung dicht umschließenden Umbeutel gesteckt werden und durch Strahlung sterilisiert werden.

Durch die verkürzte Baulänge ergibt sich eine besonders einfache Verpackungsform des Verpackungskörpers 20, wobei auch hier nicht eine fluchtende Gegenüberlage der Sperrnoppen 11, 12 notwendig ist, sondern die Sperrnoppen 11, 12 können auch in einer horizontalen Linie gegeneinander versetzt angeordnet werden, um so einen Verteilungseingriffzu ermöglichen.

Selbstverständlich ist es möglich, einen in den Dimensionen verringerten Verpackungskörper 20 auch mit paarweise angeordneten Sperrnoppen 11, 12 auszubilden, wie dies anhand der Figuren 1 ff. geschildert wurde.

Ansonsten gelten für die gleichen Teile die gleichen Bezugszeichen.

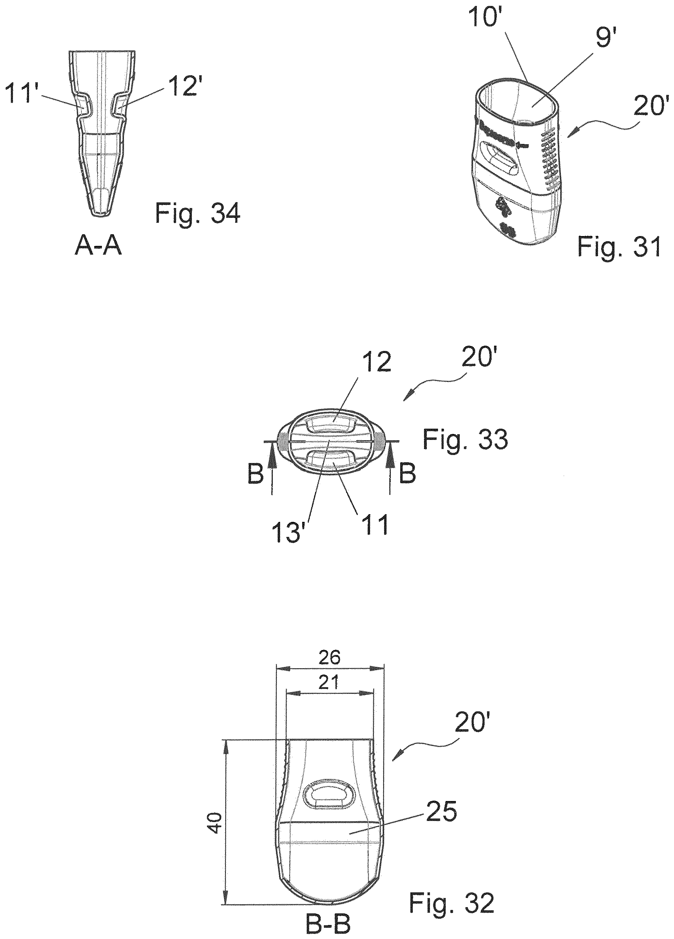

Die Figuren 31 bis 34 zeigen die gleiche Verpackung nach Figur 21 bis 30 im geöffneten Zustand, also in dem Zustand, in dem die Öffnung 9 aufgeweitet ist und in die Öffnung 9' und den umlaufende Rand 10'in einer angenäherten erweiterten Oval- oder Rundform umgeformt wurden. Dadurch hat sich der Verschlussspalt 13' gemäß Figur 33 geöffnet und es können durch den geöffneten Verschlussspalt 13' ein oder mehrere Verpackungsgegenstände 17 in den sich darunter anschließenden Verpackungsraum 25 eingefüllt werden.

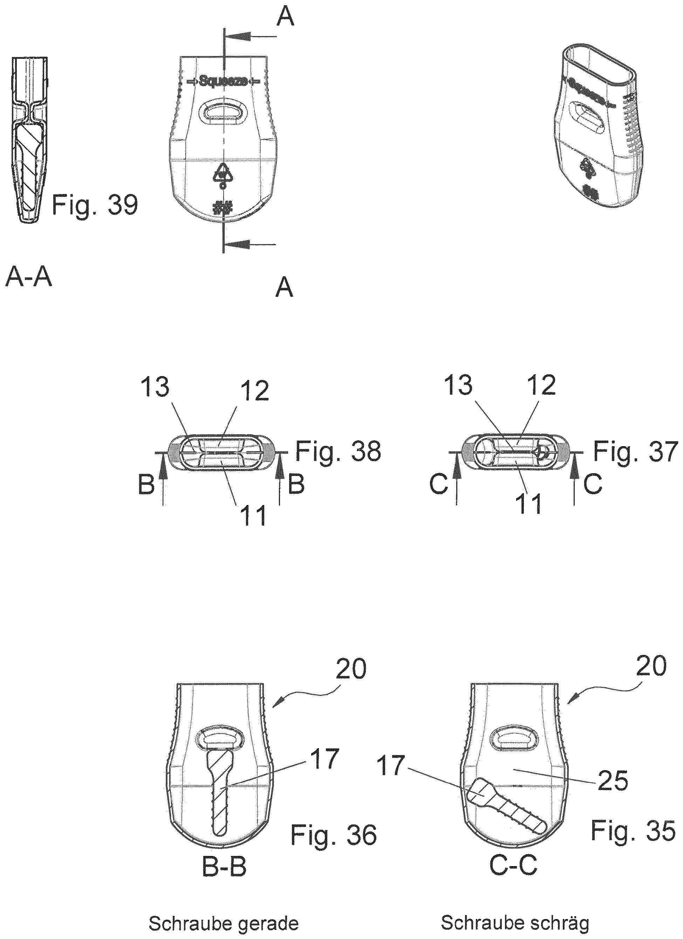

Die Figuren 35 bis 45 zeigen die gleiche Verpackung nach den Figuren 26 bis 34 bei der Aufnahme eines Gegenstandes 17, wobei die Figuren 35 und 36 zeigen, dass der Gegenstand 17 in beliebiger Weise im Verpackungsraum 25 liegen kann, und dass auch mehrere Gegenstände im Verpackungsraum 25 als Schüttgut aufgenommen werden können.

Die Figuren 37 und 38 zeigen den jeweils geschlossenen Verschlussspalt 13, wobei die Figur 39 eine lagengerechte Anordnung des Gegenstandes 17 zeigt, der sich mit seinem oberen Teil an der Unterseite der Sperrnoppen anlegt, worauf die Erfindung jedoch nicht beschränkt ist. Der Gegenstand kann jede beliebige Lage im Verpackungsraum 25 einnehmen.

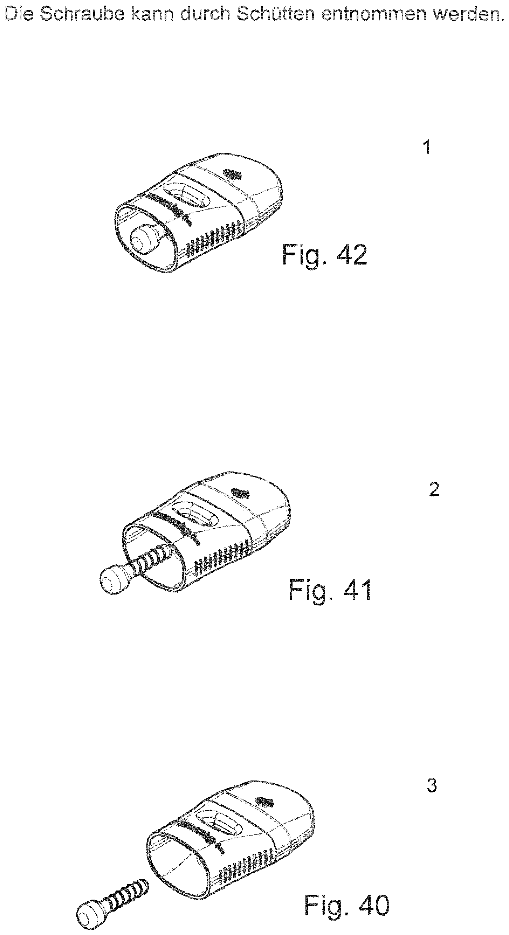

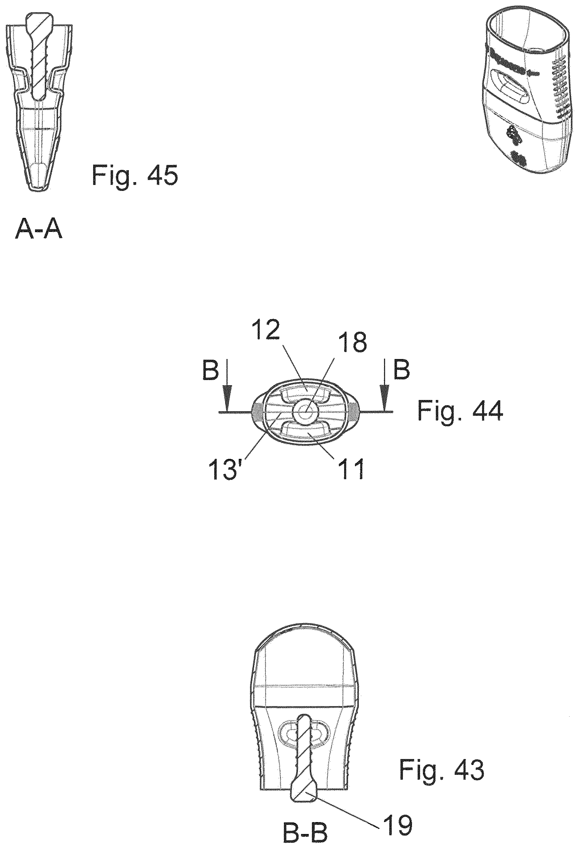

Die Figuren 40 bis 45 zeigen das schrittweise Einführen eines solchen Gegenstandes 17, wobei sich ein Durchtrittskanal 18 zwischen den Sperrnoppen bildet, durch den der Gegenstand 17 gemäß Figur 43 eingeführt werden kann, wenn die einander gegenüberliegenden Sperrnoppen 11, 12 geöffnet sind und ein erweiterter Verschlussspalt 13' vorhanden ist. Dies ist in Figur 45 ebenfalls dargestellt.

Vorteil der vorliegenden Erfindung ist demnach, dass eine elastisch aufweitbare sterilisierbare taschenförmige Schüttverpackung für einen oder mehrere Gegenstände vorhanden ist, der an seiner Stirnseite verschlossen werden kann und der sterile Gegenstände aufnehmen kann, ohne dass die Gefahr der Kontamination dieser Gegenstände durch unabsichtliche Berührung besteht, wie es beim Stand der Technik der Fall war.

Zudem ist es möglich, den Gegenstand in der verschlossenen Verpackung zu sterilisieren, was bei der Anordnung des Verpackungskörpers nach dem Stand der Technik nicht möglich war.

Besonderer Vorteil der Erfindung ist auch, dass der Verpackungskörper 1, 20 im Bereich seiner Öffnung 9 verschweißt werden kann und mit dem dort im Verpackungsraum 25 eingeschlossenen Gegenstand sterilisierbar ist und steril bleibt, ohne dass es einer Umverpackung bedarf.

### Zeichnungslegende

- 1): Verpackungskörper
- 1'): Verpackungskörper
- 2): Vorderwand
- 3): Seitenwand
- 4): Rückwand
- 5): Bodenteil
- 6): Verschlussteil
- 7): Griffrippen
- 8): Halsbereich (von 6)
- 8'): Halsbereich (von 6)
- 9): Öffnung v 9'
- 10): Rand
- 10'): Rand
- 11): Sperrnoppe vorne)
- 12): Sperrnoppe hinten)
- 13): Verschlussspalt 13'
- 14): Beschriftung
- 15): Pfeilrichtung
- 16): Pfeilrichtung
- 17): Gegenstand, 17', 17"
- 18): Durchtrittskanal
- 19): Schraubenkopf
- 20): Verpackungskörper
- 20'): Verpackungskörper
- 21): Abstand
- 25): Verpackungsraum
- 25'): Verpackungsraum

## Patentansprüche

1. Elastisch aufweitbare Verpackung (1, 20) für kleinteilige Gegenstände (17) im medizinischen Bereich, bestehend aus einem einseitig offenen Verpackungskörper (1, 20) mit einem bodenseitig geschlossenen Verpackungsraum (25, 25'), der an seiner oberen Seite in ein elastisch aufweitbares Verschlussteil (6) übergeht, **dadurch gekennzeichnet, dass** im Übergangsbereich zwischen dem Verschlussteil (6) und dem Verpackungsraum (25, 25') ein oder mehrere Sperrnoppen (11, 12) angeordnet sind, die zwischen sich einen elastisch aufweitbaren Verschlussspalt (13, 13') bilden.

2. Verpackung nach Anspruch 1, **dadurch gekennzeichnet, dass** der Verpackungskörper (1, 20) als elastisch aufweitbarer, flacher taschenförmiger Verpackungskörper ausgebildet ist.

3. Verpackung nach Anspruch 1 oder 2, **dadurch gekennzeichnet, dass** das obere Verschlussteil (6) durch Fingerkraft elastisch aufweitbar ist.

4. Verpackung nach einem der Ansprüche 1 bis 3, **dadurch gekennzeichnet, dass** am Verschlussteil (6) mindestens zwei auf den Schmalseiten des Verpackungskörpers (1, 20) gegenüber liegend angeordnete Griffrippen (7) angeordnet sind, die gegeneinander gerichtet elastisch verformbar sind.

5. Verpackung nach Anspruch 4, **dadurch gekennzeichnet, dass** die Sperrnoppen (11, 12) zur Ausbildung des Verschlussspaltes (13) im Zwischenraum zwischen den einander gegenüberliegenden Griffrippen (7) angeordnet sind.

6. Verpackung nach einem der Ansprüche 1 bis 5, **dadurch gekennzeichnet, dass** der Verpackungsraum (25) etwa beutelförmig zur Aufnahme einer Vielzahl von Gegenständen (17) ausgebildet ist.

7. Verpackung nach einem der Ansprüche 1 bis 6, **dadurch gekennzeichnet, dass** die Sperrnoppen (11, 12) jeweils einzeln oder paarweise an den Innenseiten der Vorderwand (2) und der Rückwand (4) angeordnet sind.

8. Verpackung nach Anspruch 7, **dadurch gekennzeichnet, dass** die Sperrnoppen (11, 12) fluchtend gegenüberliegen und einen geraden Verschlussspalt (13) bilden.

9. Verpackung nach Anspruch 7, **dadurch gekennzeichnet, dass** die Sperrnoppen (11, 12) zueinander auf Lücke versetzt sind und einen labyrinthartigen Verschlussspalt (13) bilden.

10. Verpackung nach einem der Ansprüche 1 bis 9, **dadurch gekennzeichnet, dass** die Sperrnoppen (11, 12) jeweils einzeln oder paarweise nur an der Innenseite der Vorderwand (2) oder der Innenseite der Rückwand (4) angeordnet sind.

11. Verpackung nach einem der Ansprüche 1 bis 10, **dadurch gekennzeichnet, dass** der Übergang vom ovalen Verpackungsraum (25) in den sich oben anschließenden ovalen Halsbereich (8) des Verschlussteils (6) über eine bogenförmige konvexe Kontur erfolgt, sodass der Halsbereich (8) im Querschnitt gegenüber dem Verpackungsraum (25) in seiner Breite verringert ist.

12. Verpackung nach Anspruch 11, **dadurch gekennzeichnet, dass** der Halsbereich (8) eine obere, unverformte ovale Öffnung (9) ausbildet, die durch einen umlaufenden Rand (10) definiert ist und dass die Öffnung (9) elastisch aufweitbar ist.

13. Verpackung nach Anspruch 11 oder 12, **dadurch gekennzeichnet, dass** die ovale Öffnung (9) des Halsbereichs (8) durch eine Schweißung oder Klebung oder Siegelung verschließbar ist.

14. Verpackung nach einem der Ansprüche 1 bis 13, **dadurch gekennzeichnet, dass** der Verpackungskörper (1, 20) in einem Stück im Hohlblasverfahren hergestellt ist und aus einem elastischen Kunststoff, bevorzugt einem TPU-Kunststoff, besteht.

15. Verpackung nach einem der Ansprüche 1 bis 14, **dadurch gekennzeichnet, dass** sie aus einem Kunststoff mit einer Härte im Bereich von 45 bis 95 shore-A besteht und dabei besonders bevorzugt eine Härte von 85 shore-A +- 5 aufweist.

## Geänderte Patentansprüche

### Geänderte Patentansprüche gemäss Regel 137(2) EPÜ.

1. Elastisch aufweitbare sterilisierbare Schüttverpackung¹ (1, 20) für kleinteilige Gegenstände (17) im medizinischen Bereich, bestehend aus einem einseitig offenen Verpackungskörper (1, 20) mit einem bodenseitig geschlossenen Verpackungsraum (25, 25`), der an seiner oberen Seite in ein elastisch aufweitbares Verschlussteil (6) übergeht, **dadurch gekennzeichnet, dass** im Übergangsbereich zwischen dem Verschlussteil (6) und dem Verpackungsraum (25, 25') ein oder mehrere Sperrnoppen (11, 12) angeordnet sind, die den Verpackungsraum nach oben hin begrenzen² und die zwischen sich einen elastisch aufweitbaren Verschlussspalt (13, 13') bilden.

2. Schüttverpackung nach Anspruch 1, **dadurch gekennzeichnet, dass** der Verpackungskörper (1, 20) als elastisch aufweitbarer, flacher taschenförmiger Verpackungskörper ausgebildet ist.

3. Schüttverpackung nach Anspruch 1 oder 2, **dadurch gekennzeichnet, dass** das obere Verschlussteil (6) durch Fingerkraft elastisch aufweitbar ist.

4. Schüttverpackung nach einem der Ansprüche 1 bis 3, **dadurch gekennzeichnet, dass** am Verschlussteil (6) mindestens zwei auf den Schmalseiten des Verpackungskörpers (1, 20) gegenüber liegend angeordnete Griffrippen (7) angeordnet sind, die gegeneinander gerichtet elastisch verformbar sind.

5. Schüttverpackung nach Anspruch 4, **dadurch gekennzeichnet, dass** die Sperrnoppen (11, 12) zur Ausbildung des Verschlussspaltes (13) im Zwischenraum zwischen den einander gegenüberliegenden Griffrippen (7) angeordnet sind.

6. Schüttverpackung nach einem der Ansprüche 1 bis 5, **dadurch gekennzeichnet, dass** der Verpackungsraum (25) etwa beutelförmig zur Aufnahme einer Vielzahl von Gegenständen (17) ausgebildet ist.

7. Schüttverpackung nach einem der Ansprüche 1 bis 6, **dadurch gekennzeichnet, dass** die Sperrnoppen (11, 12) jeweils einzeln oder paarweise an den Innenseiten der Vorderwand (2) und der Rückwand (4) angeordnet sind.

8. Schüttverpackung nach Anspruch 7, **dadurch gekennzeichnet, dass** die Sperrnoppen (11, 12) fluchtend gegenüberliegen und einen geraden Verschlussspalt (13) bilden.

9. Schüttverpackung nach Anspruch 7, **dadurch gekennzeichnet, dass** die Sperrnoppen (11, 12) zueinander auf Lücke versetzt sind und einen labyrinthartigen Verschlussspalt (13) bilden.

10. Schüttverpackung nach einem der Ansprüche 1 bis 9, **dadurch gekennzeichnet, dass** die Sperrnoppen (11, 12) jeweils einzeln oder paarweise nur an der Innenseite der Vorderwand (2) oder der Innenseite der Rückwand (4) angeordnet sind.

11. Schüttverpackung nach einem der Ansprüche 1 bis 10, **dadurch gekennzeichnet, dass** der Übergang vom ovalen Verpackungsraum (25) in den sich oben anschließenden ovalen Halsbereich (8) des Verschlussteils (6) über eine bogenförmige konvexe Kontur erfolgt, sodass der Halsbereich (8) im Querschnitt gegenüber dem Verpackungsraum (25) in seiner Breite verringert ist.

12. Schüttverpackung nach Anspruch 11, **dadurch gekennzeichnet, dass** der Halsbereich (8) eine obere, unverformte ovale Öffnung (9) ausbildet, die durch einen umlaufenden Rand (10) definiert ist und dass die Öffnung (9) elastisch aufweitbar ist.

13. Schüttverpackung nach Anspruch 11 oder 12, **dadurch gekennzeichnet, dass** die ovale Öffnung (9) des Halsbereichs (8) durch eine Schweißung oder Klebung oder Siegelung verschließbar ist.

14. Schüttverpackung nach einem der Ansprüche 1 bis 13, **dadurch gekennzeichnet, dass** der Verpackungskörper (1, 20) in einem Stück im Hohlblasverfahren hergestellt ist und aus einem elastischen Kunststoff, bevorzugt einem TPU-Kunststoff, besteht.

15. Schüttverpackung nach einem der Ansprüche 1 bis 14, **dadurch gekennzeichnet, dass** sie aus einem Kunststoff mit einer Härte im Bereich von 45 bis 95 shore-A besteht und dabei besonders bevorzugt eine Härte von 85 shore-A +- 5 aufweist.
